(19) Europäisches Patentamt
European Patent Office
Office européen des brevets

(11) **EP 2 434 270 B1**

(12) **EUROPEAN PATENT SPECIFICATION**

(45) Date of publication and mention
of the grant of the patent:
**26.06.2013   Bulletin 2013/26**

(51) Int Cl.:
**G01M 15/10** $^{(2006.01)}$

(21) Application number: **10193141.8**

(22) Date of filing: **30.11.2010**

(54) **Method and system for determining a mass emission rate of a pollutant contained in the exhaust of a mobile equipment**

Verfahren und System zur Bestimmung der Massenemissionsrate eines Schadstoffes im Abgas von mobilen Gerätschaften

Procédé et systeme pour la détermination du taux d'émission massique d'un polluant dans le gaz d'échappement d'un équipment mobile

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2010   EP 10180149**

(43) Date of publication of application:
**28.03.2012   Bulletin 2012/13**

(73) Proprietor: **MAHA Maschinenbau Haldenwang GmbH & Co. KG**
**87490 Haldenwang (DE)**

(72) Inventor: **Anyon, Peter**
**4561 Queensland (AU)**

(74) Representative: **MERH-IP**
**Matias Erny Reichl Hoffmann**
**Paul-Heyse-Strasse 29**
**80336 München (DE)**

(56) References cited:
**WO-A1-03/038393       WO-A1-2005/085793**
**US-A- 5 639 957**

**Description**

1 Technical Field

The present invention relates to a method and a system for determining the rate, for example in grams per kilometer or in grams per kilowatt hour, at which products of combustion are emitted from mobile equipment powered by a combustion-based energy source while being propelled on a static medium such as a road or on a dynamometer.
The invention has particular but not exclusive application to the measurement of both gases and/or fine particles in the exhaust stream of a combustion process utilized to generate motive power for propelling mobile equipment. For illustrative purposes, the invention will be described with reference to the preferred application of the invention, but without limiting its scope in any way thereto, i.e. measuring mass emission rates for internal combustion engines operating in a motor vehicle.

2 Background of Invention

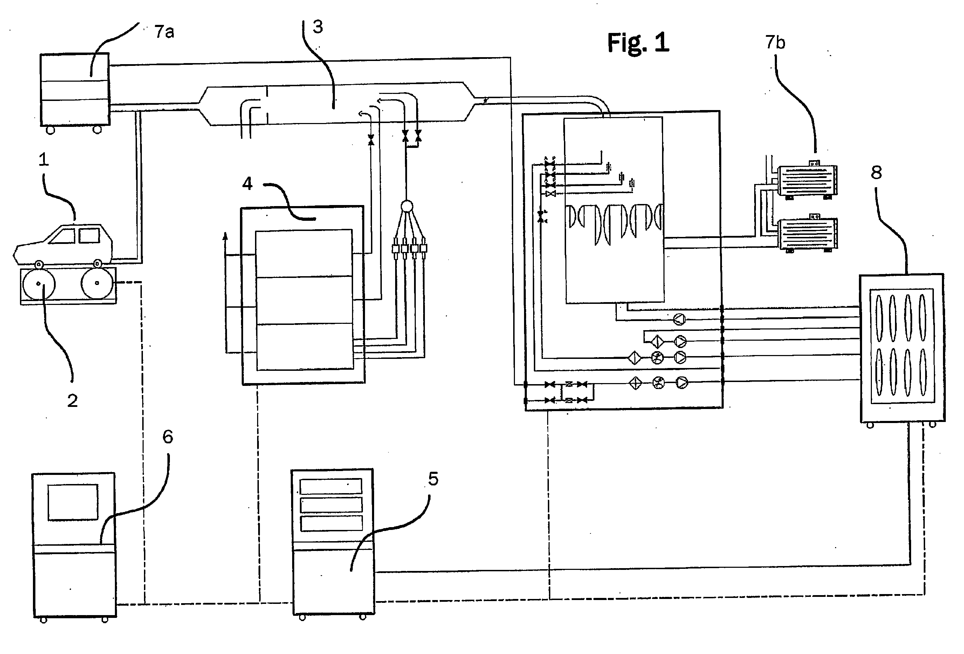

Over recent years, health and environmental authorities around the world have placed increased emphasis on reducing the adverse health impacts of airborne particulate and gaseous pollutants.
Research has shown that exhaust emissions from motor vehicles and other equipment using internal combustion engines are a dominant source of many pollutants of concern. Most notable of these are fine particulates and a range of chemical substances that combine to precipitate the formation of photochemical smog.
Particulate emissions, most commonly from internal combustion engines, have been widely acknowledged to be one of the most significant pollutant health threats to humans. These particulate emissions, comprising a mixture of very finely divided carbonaceous matter, sulfates and highly toxic compounds are often carcinogenic and can affect the neurological and reproductive systems of humans. Internal combustion engines are also a major source of gaseous pollutants including a range of hydrocarbons and oxides of nitrogen. These gases may individually or in combination adversely affect human health, especially in relation to the incidence or exacerbation of respiratory ailments.
Although research-grade laboratory instrumentation and test methods for measuring engine emissions have been developed, they are generally very expensive and the processes are time-consuming, making them unsuitable for use or adaptation to the low-cost, high volume measurements required to rapidly assess the emissions performance of in use vehicles.
New motor cars and light commercial vehicles, and the engines used in heavy goods vehicles, are certified to a suite of regulations, using standardized test methods. A schematic example of a prior art testing facility is illustrated in Fig. 1. The most common method of measuring exhaust emissions of a vehicle is testing on a dynamometer 2, where the total exhaust flow is captured and entrained with an amount of filtered air (cf. filter 7a) into a dilution tunnel 3, in which the total exhaust/ambient air flow is drawn through a critical flow venturi by a powerful extraction blower 7b. The testing facility further comprises a particle measuring cabinet 4 with a particle mass controller, solid particle counter and a flame ionization detector, and further an exhaust gas measuring equipment 5, a dynamometer main computer 6, and a sample cabinet 8. The total cost of such a facility is typically in the range €2million to €10million, with a typical cost per test of many thousands of Euros and a delay of up to three hours or even more to generate a result.
For periodic testing of in-use vehicles a similar facility, with lower grade dynamometer and instrumentation, can be used where the regulating agency wishes to obtain results that can be directly correlated against the original certification emission limits. Such a facility for testing both light and heavy-duty vehicles is also expensive - typically in the region €0.6 to €1.0 million, excluding the building structure and site costs.
Although this method of testing gives results which most closely match emission rates during typical on-road driving, the cost of establishing such facilities, together with the time involved in restraining the vehicle on the dynamometer and connecting the exhaust collection homes to the vehicle, deters most regulators from using this method.
Alternatively, on-board portable emission measuring systems (PEMS) with total exhaust flow measurement, partial exhaust sampling, proportional sample flow management and scaled-down versions of laboratory instrumentation can be mounted on a vehicle to measure emissions while the vehicle is being operated in typical traffic situations. Again, the cost of the equipment is considerable and its use requires an installation time typically exceeding two hours. The cost of this type of equipment is generally greater than €200,000.
A very significant component of the cost of the systems described above is the control or direct measurement of the total exhaust flow. Measurement of the raw or diluted exhaust flow is essential in these systems in order to generate a test result in terms of grams per kilometre or grams per kilowatt hour of each pollutant.
While these costs may be acceptable for vehicle certification and many research and development activities, they are in most cases prohibitively high for other vitally important applications, even the lower grade facilities are not economically viable for other vitally important applications, such as:

- emissions inventory studies, or

- periodic in-use vehicle test-and-repair programs to quickly identify and rectify high polluting vehicles.

In view of the above problems of the prior art testing methods, the present invention seeks to provide a method of quickly and less costly measuring mass emission rates of pollutants emitted by road vehicles, without the complexity and cost of equipment such as that described above. It is a further object of the invention to provide compact test equipment that can be quickly installed on the vehicle.

US 5,639,957 discloses a method and apparatus for measuring the quantity of an ingredient gas in the exhaust gas from a motor vehicle wherein approximate actual exhaust flow rate values $QEa(t)$ are calculated using a polynomial curve fit method on the calculated theoretical flow rate values $QEi(t)$.

WO 03/038393 A1 discloses an approach to deriving an exhaust flow rate comprising the measurement of variables such as air flow, fuel H:C ratio, $CO_2$, CO and HC and deriving a measure of exhaust flow rate based on a novel formula. The rate so derived is less sensitive than known procedures to fluctuations in emissions values and/or transport times between engine the engine and exhaust.

WO 2005/085793 A1 discloses method and to a device which is used to determine the mass emission of an exhaust gas component in a vehicle, comprising a measuring and evaluation unit used to determine the concentration of at least one exhaust gas component. The exhaust gas line of the measuring and evaluation unit is connected to the waste gas train of the vehicle and comprises a plug-in or a wireless data connection to form an OBD interface of the vehicle. The temporal mass emission of the exhaust gas components can be determined from the concentration signal and a suction air mass signal and/or fuel consumption signal which is provided by the OBD interface.

A method of estimating a mass emission rate of a combustion product contained in the exhaust of mobile equipment powered by a combustion-based energy source is proposed. The combustion product may include particulate or gaseous combustion products, also referred to as pollutants. In particular, the mobile equipment might be a motor vehicle, but the proposed method is also applicable to ships, trains etc.

According to an aspect of the invention, the method comprises the step of measuring a combustion product concentration contained in the exhaust of the mobile equipment for a plurality of time intervals. Compact analyzers capable of measuring the combustion product concentrations have been available for some time wherein a sample of the gas to be analyzed is obtained by inserting an exhaust probe into the vehicle's exhaust pipe and connected with the analyzer via a flexible hose. For each combustion product/ pollutant for which the mass emission rate in the exhaust stream needs to determined, the respective concentration in the exhaust stream is determined. Multi-functional analyzers may be used and are known in the prior art, e.g. 4-gas or 5-gas analyzers that can determine the concentrations of four or five different pollutants quasi-simultaneously. Thus, the proposed method allows for a simultaneous estimation of the mass emission rate of multiple combustion products.

The method further comprises the step of determining a mass of the mobile equipment. For on-road testing of a vehicle, vehicle mass may be the actual weighed on-road mass of the vehicle. The method further comprises the step of measuring a velocity of travel for the plurality of time intervals during a test cycle. Preferably, whilst in motion, the velocity of travel is continuously monitored and recorded at a predetermined frequency.

The method further comprises the step of estimating a volumetric exhaust flow rate for the plurality of time intervals during the test cycle based on the determined mass and the velocity of travel; and calculating the mass emission rate of the pollutant as the product of the measured combustion product concentration and the determined volumetric exhaust flow rate. In other words, instead of measuring the volumetric exhaust flow rate directly, it is determined as a function of vehicle motion using easy and less costly-to-measure variables, such vehicle mass and vehicle speed. The vehicle mass and speed are related to the vehicle propulsion load and power, which in turn determine the required fuel consumption and thus, as a consequence, the volumetric rate of combustion gas generation caused by the consumed fuel.

Thus, according to another aspect of the invention, in the step of estimating a volumetric exhaust flow rate for the plurality of time intervals, an instantaneous power required to propel the mobile equipment may be calculated for the plurality of time intervals based on the determined mass and the velocity of travel; and the calculated instantaneous power may be used to calculated the instantaneous mass fuel consumption rate. However, the volumetric exhaust flow may also be directly calculated using vehicle mass and speed without the intermediate step of calculating the instantaneous power.

According to a further aspect of the invention, the method may further comprise the step of measuring a concentration of an indicator combustion product contained in the exhaust of the mobile equipment during the time interval; wherein the determined indicator combustion concentration may be used to correct the estimated volumetric exhaust flow rate by a factor calculated as the ratio of the measured indicator combustion concentration in the exhaust stream versus a concentration of the indicator combustion product that would result from stoichiometric combustion. The indicator combustion product may be a different combustion product than the combustion product for which the mass emission rate is to be estimated. Preferably, carbon dioxide ($CO_2$) may be used as the indicator combustion product for which the concentration is additionally measured. In this case, the determined CO2 concentration may be used to correct the estimated volumetric exhaust flow rate by a factor calculated as the ratio of the measured carbon dioxide concentration in the exhaust stream versus a concentration of carbon dioxide in the exhaust stream that would result from stoichiometric combustion. This step further increases the estimation accuracy in case the combustion engine does not operate on stoichiometric combustion, as it is typically the case for diesel engines.

According to a further aspect of the invention, the method may further comprise the step of determining a power-specific fuel consumption factor, wherein the power specific fuel consumption factor is determined either using an estimated constant brake specific fuel consumption value (BSFC) or using a BSFC value determined based on the measured engine speed and a determined engine torque during the time interval. The power-specific fuel consumption factor further increases the accuracy of estimating the volumetric exhaust flow rate since the volumetric exhaust flow rate is not always directly proportional to vehicle speed or power output but varies with both engine revolutions and engine torque.

According to a further aspect of the invention, empirical or measured factors for at least one of an aerodynamic loss, a tyre rolling loss, a transmission loss or a loss due to accessory power loads may be used to determine a tractive power required to propel the mobile equipment at the determined speed. Such factors further increase the estimation accuracy by taking the tractive loads into accounts resulting from one or several of these factors. Preferably the factors for the above tractive loads are also estimated based on vehicle mass, only.

According to a further aspect of the invention, the output of a mass-based accelerometer aligned with the longitudinal axis of travel may be used to determine the net tractive load due to the algebraic sum of a gradient and a longitudinal acceleration or deceleration. The gravitational component of total tractive load may also be isolated from the net acceleration load by algebraically subtracting the longitudinal acceleration forces due to rate of change of longitudinal velocity, measured using a GPS sensor or other speed sensing means to measure velocity at a predetermined output frequency.

According to a further aspect of the invention, the method may further comprise the step of integrating the estimated mass emission rates over the plurality of time intervals of the test cycle together with the actual distance travelled during the plurality of time intervals to calculate the mass of the combustion product generated per kilometer of travel.

According to a further aspect of the invention, the method may further comprise the step of integrating a derived average engine power during the test cycle with the estimated mass emission rates over the plurality of time intervals to calculate the mass of the pollutant generated per used energy unit.

According to a further aspect of the invention, the mass emission rate may be estimated based on the driving of the vehicle on a normal road or on a dedicated test track. Alternatively, according to a further aspect of the invention, the vehicle may be tested on a dynamometer for estimating the mass emission rate, wherein different driving conditions are simulated using dynamometer load curves.

According to a further aspect of the invention, a system is proposed for estimating a mass emission rate of a particulate or gaseous pollutant contained in the exhaust of a mobile equipment powered by a combustion-based energy source. The system comprises an emission analyzer device for measuring concentration rates of one or multiple gaseous and particulate combustion products; a vehicle velocity sensor; and a sample hose transmitting an exhaust sample to the device for measuring the concentration rates of the gaseous and particulate emissions; and a further processing unit configured to conduct the method of estimating a mass emission rate as described above. The processing unit may be integrated with the emission analyzer or provided as a separate device, for example as a mobile computer. According to a further aspect of the invention, the system may further comprise an engine speed sensor. The vehicle velocity sensor and/ or the engine speed sensor may be provided separately as mobile devices provided with the above system. Alternatively, the vehicle's built-in speed sensor and engine speed sensor may be used and accessed via the vehicle's OBD (on-board diagnostic) data interface. According to a further aspect of the invention, the system for estimating a mass emission may be placed inside the vehicle during a measurement period for estimating the mass emission rate.

It is a particular advantage of the invention that it is capable of estimating a mass emission rate for one or multiple pollutants in the exhaust stream of mobile equipment such as a vehicle without the need for capturing the total exhaust flow using an expensive testing facility. Instead of measuring the volumetric exhaust flow rate, the proposed method relies only on simple-to-measure variables such as speed and mass of the mobile equipment. It is a further advantage, that the mass emission rate can be determined in a real-time situation while driving the vehicle on the road or on a dedicated test track.

Summarizing the above, a method of measuring mass emission rates of pollutants contained in the exhaust of motor vehicles is disclosed which includes continuously measuring the speed and acceleration at which a vehicle of known inertial mass is operating, while simultaneously measuring the concentration of pollutants in the vehicle's exhaust stream, and preferably also the engine's rate of revolutions. By continuously calculating the energy required to maintain the vehicle's speed and acceleration and preferably linking these factors with a predetermined rate of fuel consumption associated with the instantaneous engine speed-engine power combination, the volumetric rate of exhaust flow may be calculated. Multiplying the volumetric rate of exhaust flow by the simultaneous mass concentration of pollutants in the exhaust may deliver the mass emission rate for each pollutant per unit time. Integrating the measured speed and calculated energy profiles on a time basis may then allow calculation of emission rates in grams per kilometer and/or grams per kilowatt-hour for each pollutant monitored.

4 Description of Embodiments

The invention is explained below in an exemplary manner with reference to the accompanying drawings, wherein

Fig. 1        shows a schematic example of a prior art certification grade emission testing facility;

Fig. 2        shows a schematic example of a system for estimating the mass emission rate according to an embodiment of the invention;

Fig. 3        shows another schematic example of a system for estimating the mass emission rate according to an embodiment of the invention;

Fig. 4        shows a flow diagram of steps involved in estimating the mass emission rate according to an embodiment of the invention;

Fig. 5        shows a flow diagram of steps involved in estimating the volumetric exhaust flow according to an embodiment of the invention;

Fig. 6        illustrates how fuel consumptions varies with both engine revolutions and mean cylinder pressure;

Figs.7A and   7B illustrate schematically the flows and CO2 concentrations for stoichiometric combustion gaseous matter and 100% excess air combustion; and

Fig. 8        shows a flow diagram of steps involved in estimating the mass emission rate according to another embodiment of the invention.

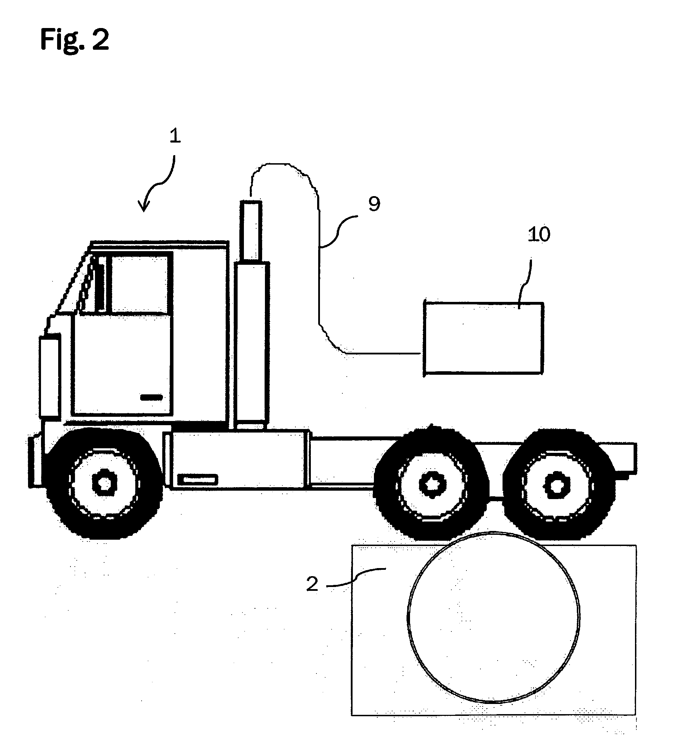

In cases where the testing facility operator wishes to use a dynamometer, but does not wish to carry the expense or the time delays associated with direct measurement of exhaust flow (see Figure 1), this invention allows the exhaust flow to be approximated, utilizing measurements of other physical parameters such as speed and/ or dynamometer torque. In this type of facility the only equipment connected to the vehicle exhaust is a simple probe and hose to convey a small sample of the exhaust to the instruments measuring the range of pollutants of interest (see Fig. 2).

When it is desired to avoid the use of both a dynamometer and exhaust flow measurement, this invention provides a method of accurately estimating emission rates in units of grams per kilometer or grams per kilowatt-hour by driving the vehicle on the road or on a dedicated test track. In this case the calculations may utilize a larger set of parameters, all of which are can be linked to vehicle mass and velocity.

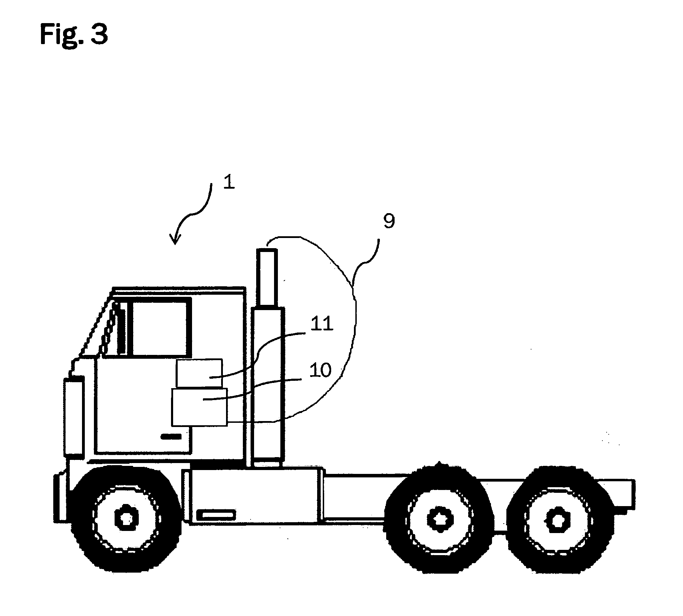

Fig. 3 illustrates the simplicity of this approach. In this example the full benefits of the invention are exploited, using a very compact instrument 10 capable of measuring gaseous and particulate emissions, together with an electronic velocity sensor 11. A second sensor (not shown) for measuring the rate of engine revolutions, may optionally be incorporated in the system to enhance the accuracy of estimated emission rates. The emission analyzer, velocity sensor and engine speed sensor can be conveniently placed inside the vehicle while testing. The emission analyzer is further connected to a mobile computer (not shown) configured to generate real-time output and storage of the measured parameters on a set frequency and to estimate the emission rates based on the proposed estimation method as illustrated below in exemplary embodiments.

An additional benefit of the method described in this invention is that the test equipment required can be low-cost, very compact, and installed on the vehicle ready for testing in typically less than five minutes.

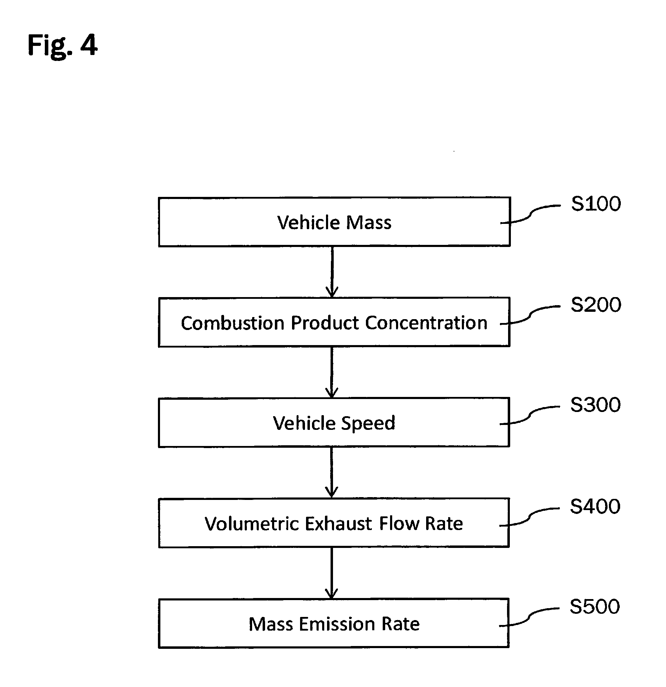

Fig. 4 illustrates a flow diagram of steps according to an embodiment of the invention. In step S100, the inertial mass of the vehicle being tested is measured and recorded. For dynamometer testing as shown in Fig. 2, the Vehicle Mass is measured as the mean of the plated empty (tare) and fully laden (GVM) mass of the vehicle being tested.

$$Mv = (Tare + GVM)/2 \qquad (kg)$$

For on-road testing (cf. Fig. 3), Vehicle Mass is the actual weighed on-road mass of the vehicle. The following measurements of the combustion product concentrations and vehicle speed are conducted for a test cycle during which the vehicle is driven either on a dynamometer (cf. Fig. 2) or on any fixed plane such as a road, either on a random driving pattern or a predetermined speed/time profile (cf. Fig. 3).

In step S200, whilst in motion, a sample of exhaust from the raw exhaust stream is withdrawn and distributed with the hose 11 to instruments 10 capable of continuously measuring the combustion product concentration of each of the pollutants of interest contained in the exhaust of the vehicle 1. The combustion product concentrations are measured for a plurality of time intervals using real-time gaseous and particle analyzers 10 as shown in Fig. 2 and Fig. 3. Thus, for each pollutant or combustion product for which the mass emission rate is to be estimated, a time series of the measured product concentrations is generated over the complete test cycle. Such real-time gaseous and particle analyzers 10 as shown in Fig. 2 and Fig. 3 are known from the prior art and provide measurements of mass concentration ($g/m^3$), or units which can be readily converted to $mg/m^3$.

Whilst in motion, the speed of the vehicle is continuously monitored and at a predetermined frequency/ time intervals, the vehicle velocity is measured and recorded (Step S300).

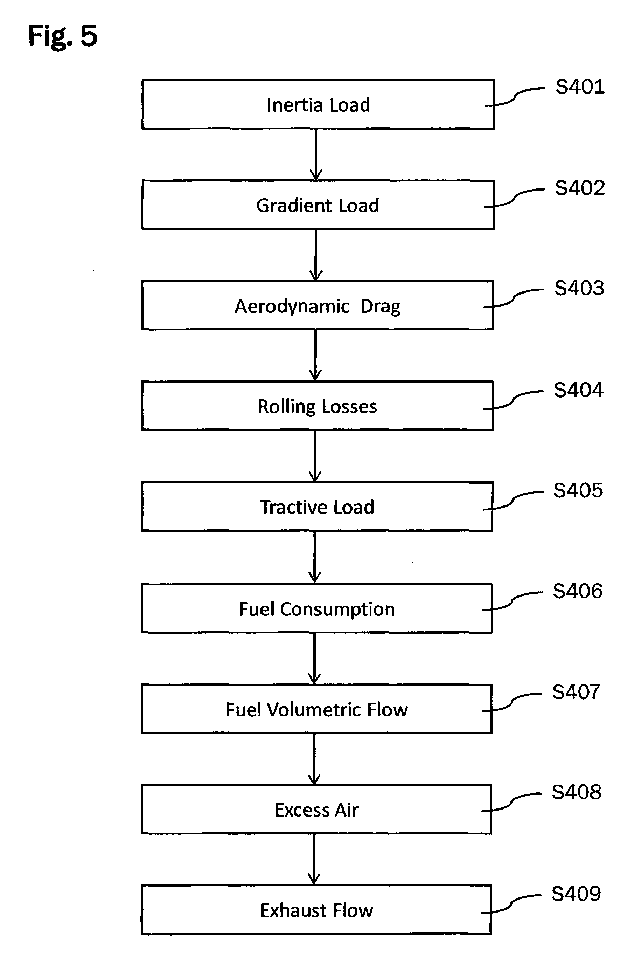

In step S400, the volumetric exhaust flow rate for the plurality of time intervals is estimated based on the determined mass and the velocity of travel. In other words, the volumetric exhaust flow rate is determined as a function of vehicle motion, since the volumetric rate of combustion gas generation is caused by fuel consumption based on engine power and speed which is dependent on vehicle propulsion load and power, which can be derived directly from vehicle mass and vehicle speed. The estimation of volumetric exhaust flow rate is described in more detail further below based on Fig. 5.

In step 500, for each pollutant being measured, the mass emission rate of the pollutant (or the pollutants) are calculated as the product of the measured combustion product concentration, converted to grams per unit volume, and the determined volumetric exhaust flow rate, corrected to normal temperature and pressure.

Integrating this result for each frequency period, together with the actual distance travelled during each matching frequency period (determined from the instantaneous velocity measurement) allows simple calculation of the mass of each pollutant generated per kilometer of travel. Similarly, by integrating the derived average engine power during the test at each frequency period with the mass of each pollutant emitted during each frequency period allows a second calculation of the mass of each pollutant generated per kilowatt hour.

Fig. 5 is a flow diagram of steps describing in more detail the estimation of volumetric exhaust flow rate. The volumetric exhaust flow rate is determined as a function of vehicle motion, ultimately based on the vehicle's mass and speed profile during the test cycle. Preferably, other factors are additionally used in order to increase the estimation accuracy.

The volumetric rate of combustion gas generation is caused by fuel consumption based on engine power and speed which is dependent on vehicle propulsion load and power, which can therefore be derived directly from vehicle mass and vehicle speed.

According to this embodiment, it is proposed to calculate the vehicle propulsion load and power using the vehicle's mass and speed, then to calculate the fuel consumption based on engine power and speed. Finally, the volumetric rate of combustion gas generation can be estimated based on the determined fuel consumption.

The various calculation steps below are constructed from a series of elements, each of which calculate the individual components of the total force (or "tractive load") required to propel a vehicle at any given speed/acceleration/ gradient condition. These components take account of: Vehicle Mass, Acceleration/Deceleration, Gradient, Aerodynamic Drag (frontal area, drag coefficient, air density), Rolling Losses (tyre hysteresis), Transmission Losses and Accessory (e.g., air conditioning) Losses. It is noted that in other embodiments of the invention (not shown) that require less accuracy, not all of the above components have to be taken into account. The method according to this embodiment may be used either to calculate the actual tractive load during on-road driving, or alternatively using a dynamometer wherein dynamometer load curves are set to ensure that the dynamometer applies appropriate loads under all driving conditions.

An important consideration in developing the estimation method is to avoid the need for testing personnel to make judgments or estimates of variable factors, such as frontal area and drag coefficients. Individual factors are discussed below. In the following, unit of measurement are written in square brackets.

In step S401, the Inertia Load (IL) is calculated. Inertia load in [N] is simply Vehicle Mass (Mv) multiplied by acceleration. Acceleration is calculated as:

$$\text{Accn} = (V0\text{-}V1) \times F, \text{ in } [m/s2]$$

Where:

V0 = current speed [m/s], V1 = last measured speed [m/s]), F= measurement frequency [Hz]

Hence:

$$\text{Hence:} \qquad IL = Mv \times (V0-V1) \times F$$

In step S402, the tractive force attributable to gradient is calculated as the Gradient Load (GL) [N]. Although exact gradient loads are calculated using trigonometric formulas, for most typical road grades it is sufficiently accurate to calculate the load as:

$$GL = \text{Vehicle Mass} \times g \times \% \text{ grade, in [N]}$$

or: GL = Mv x 9.81 x % grade, in [N]
Where:

$g$ = gravimetric acceleration (~ 9,81 m/s$^2$)

GL may also be determined by subtracting the previously calculated horizontal plane tractive force from the total inertial force determined by multiplying the vehicle mass by the rate of acceleration recorded by the accelerometer. The grade may also be determined using a GPS or other speed sensor.
In step S403, the Aerodynamic Drag (AD) is estimated based on the frontal area (FA) and drag coefficient (DC). The frontal area (FA) and drag coefficient (DC) are calculated using empirical non-linear formulas based only on vehicle mass (Mv). In practice, the values produced by the formulas have been shown to provide reliable estimates. A suitable non-linear coefficient has been found to be the sum of a fixed value and a sinusoidal factor, as shown below.

(a) Frontal Area (FA) can either be measured and entered into data recording computer manually or calculated by a Mv -based formula:

$$\text{Direct calculation:} \qquad FA = \text{body width [m]} \times \text{body height [m], in [m}^2]$$

$$Mv \text{ -based formula: } FA = 2.6 + (7.4 \times (\sin(90 \times Mv /45000))), \text{ in [m}^2]$$

(b) Drag Coefficient (DC)

$$Mv \text{ -based formula: } DC = 0.45 + (0.55 \times (\sin(90 \times Mv /45000)))$$

(c) Air Density

$$\text{Air Density} = \text{constant} = 1.2, \text{ in [kg/m}^3]$$

This resolves to:

$$AD = 0.5 \times 1.2 \times V_0^2 \times FA \times DC, \text{ in [N] or } AD = 0.6 \times V_0^2 \times FA \times DC, \text{ in [N]}$$

where $V_0$ = velocity [m/s]

In step S404, the Rolling Losses (RL) are calculated. The dominant rolling loss is generated by tyre hysteresis/friction (transmission losses are not included as they are "upstream" of the dynamometer).

The general equation is:

$$RL = Mv \times g \times \text{tyre loss factor, in [N]}$$

where: g = gravitational acceleration = 9.81, in [m/s$^2$], Mv = Vehicle Mass, in [kg] The value used for tyre loss factor can vary with tyre construction and dynamometer roller diameter. A factor of 0.008 is widely used. In this case:

$$RL = M_v \times 9.81 \times 0.008$$

or **RL=** $M_v$ x 0.078, in [N]

Combining these elements, in simplified form the instantaneous tractive load is calculated in step S405 to be:

$$\text{Tractive Load (TL)} = AD\ [N] + RL\ [N] + IL\ [N] + GL, \text{ in } [N]$$

$$TL = (0.6*V^2*FA*DC)+(M_v*0.078)+(M_v*(V_0-V_1)*F)+(M_v*9.81*\%grade), \text{ in } [N]$$

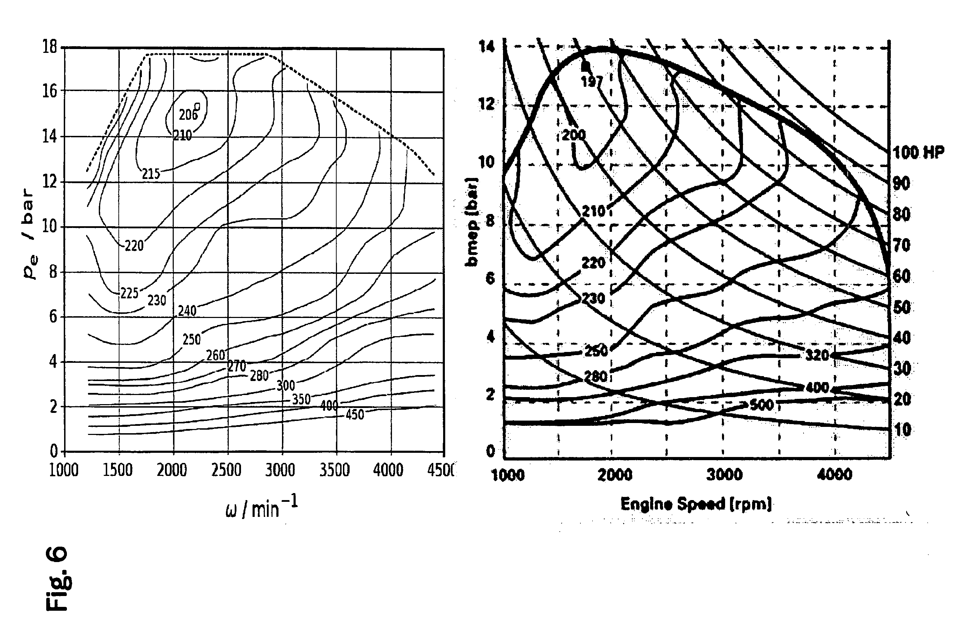

Next, the fuel consumption (FC) is calculated in step S406. In general, fuel consumption is dependent on the instantaneous power (P), where P can be calculated as

$$P = TL * V_0,$$

where TL denotes the tractive load (TL) determined in step S405 and V0 equals the vehicle velocity [m/s]. In general, fuel consumption is proportional increases with power output. However, the brake specific fuel consumption (BSFC), measured in g/kWh is not directly proportional to power output. The two "engine maps" depicted in Fig. 6 show that BSFC varies with both engine revolutions and mean cylinder pressure (proportional to torque).

As shown above, the distribution of "contours" in engine maps is highly consistent on a proportional basis, regardless of the individual engine. At high torque, medium revolutions the engine most efficiently converts fuel into mechanical energy. The converse is true for low torque, high engine speed operation. There is also only small variation in the numerical range of BSFC for a given fuel type. Gasoline engines are less efficient than diesel and hence have higher BSFC at any torque-speed combination, but the general shape of the map remains the same.

Where medium accuracy of the test result is sufficient, taking a median BSFC (for example 230 or 250 g/kWh) for diesel may provide sufficient accuracy. A corresponding figure can be selected for gasoline and other fuel types.

Where higher accuracy is needed, the use of an engine speed instrument allows an engine power curve to be generated, either on a dynamometer (if used) or by accelerating the vehicle in a low gear on the road. This curve can be used to calibrate the axes of a generic engine map such as the one shown in Fig. 6. Dividing the map into a grid of 5x5 squares defines 25 BSFC value zones of sufficient resolution to meet accuracy requirements.

During testing, the second-by-second measured engine speed and calculated power can be used to calculate second-by-second torque, allowing the vehicle's location on the engine map to be identified and the BSFC at that point recorded on a second-by-second basis in the test data. The general equations are hence:

$$\text{For medium accuracy: BSFC = Const} \qquad [g/kWh]$$

$$\text{For high accuracy:} \quad BSFC = fn(\% \text{ max torque}, \% \text{max engine speed}) \qquad [g/kWh]$$

For illustrative purposes, BSFC = 240 [g/kWh]. Hence, actual fuel consumption is given as

$$FC = P * (BSFC/3600) \ [g/s]$$

In Step S407, the calculated fuel consumption is converted to a corresponding volumetric exhaust gas flow. Firstly, using standard chemical constants, the volume of carbon dioxide is calculated generated in the current frequency period by stoichiometric combustion of the calculated fuel mass and corrected to normal temperature and pressure. Then, using the known relative volumetric proportions of nitrogen and oxygen in ambient air, the volumetric flow of ambient air is calculated which would be required for stoichiometric combustion of the calculated fuel mass burned in the current frequency period.

This is illustrated in the following assuming an example for a diesel engine: Here, the air/diesel fuel mass ratio for stoichiometric combustion is 14.6 and the air density is 1.2g/liter. Thus, 1.0 gram diesel mixes with 14.6/1.2 = 12.17 liters of air. Standard air contains approx. 21 % oxygen and 78% nitrogen (by volume). Thus, the oxygen volume in 12.17 liters of air is given as 12.17 x 0.21 = 2.55 liters; whereas the nitrogen volume in 12.17 air equals 12.17 x 0.78 = 9.49 liters. For stoichiometric combustion of 1 gram diesel fuel, the exhaust thus contains:

$$9.49 \text{ liters } N2 + 1.8 \text{ liters } CO2 = 11.29 \text{ liters total gaseous matter } [l/g],$$

and the percentage (%) of CO2 (by volume) in the exhaust is:

$$\% \ CO2 \ (1.8/11.29) \times 100 = 15.93\%$$

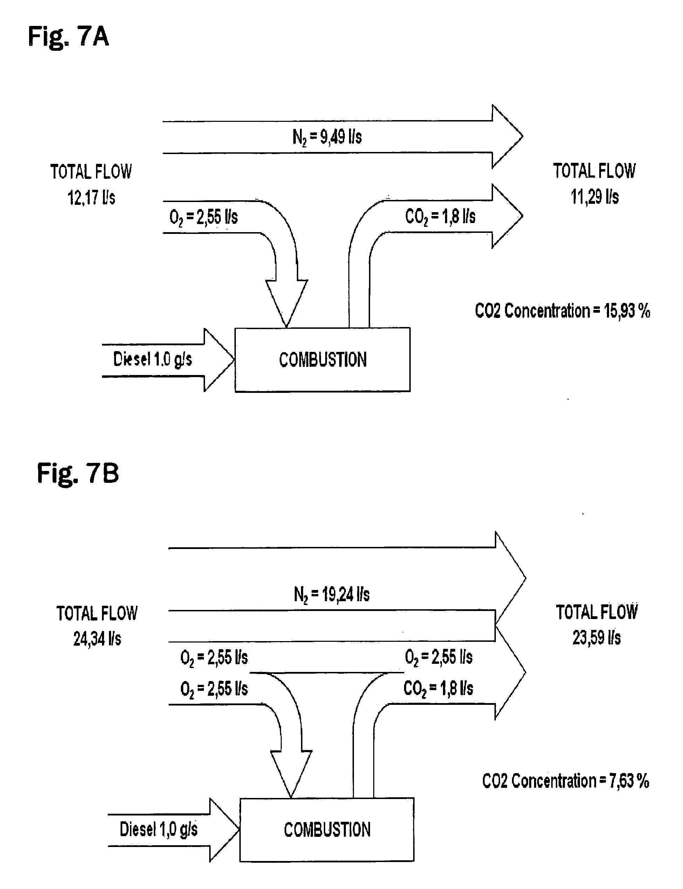

Since diesel engines do not generally operate on stoichiometric combustion, in step S408, an Excess Air (EA) factor is calculated to correct the results for non-stoichiometric combustion. At most times there is excess air (EA), which passes directly through the engine unchanged. Figs. 7A and 7B below compare flows and CO2 concentrations for stoichiometric (Fig. 7A) and for 100% excess air (Fig. 7B) combustion. From the above, where Excess Air (EA) is a numeric factor, and CO2 concentration (CO2-conc) is a decimal fraction of total flow:

$$CO2\text{-conc} = 1.8/((12.17 \times EA)-(2.55 - 1.8 \ );$$

$$(12.17 \times EA - 0.75) = 1.8/CO2\text{-conc};$$

hence EA = ((1.8/CO2conc)+0.75)/12.17

Summarizing the above, the instantaneous concentration of carbon dioxide in the exhaust stream is compared with the known concentration which would be present from stoichiometric combustion and the calculated volumetric flow of ambient air is corrected according to the ratio of theoretical carbon dioxide concentration and the actual measured carbon dioxide concentration. It is noted, that instead of CO2 also another combustion product for which the concentration ratios of stoichiometric vs. non-stoichiometric combustion is known could be used to calculate an EA factor.

Finally, in step S409, an equation can therefore be constructed to calculate the excess air, and hence the total volumetric exhaust flow (EF), based only on CO2 concentration and g/sec fuel consumption (FC), namely

$$EF = 11,29*FC*EA, \text{ in } [l/s];$$

where EA = ((1.8/CO2conc)+0.75)/12.17; FC = P (BSFC/3600) and P = TL * $V_0$, and
TL has been calculated in step S405 as TL = (0.6*V2*FA*DC)+(Mv*0.078)+(Mv *(V0-V1)*F)+(Mv *9.81 *%grade), in [N]

All of the above outputs are derived directly from vehicle mass, vehicle speed, and CO2 concentration, together with one (or optionally two) constants. The expressions can be readily programmed into a computer or microprocessor-based data management system to generate real-time output and storage of all values on a set frequency.

The gravimetric exhaust emissions can be calculated by multiplying the total volumetric exhaust flow (EF) with the provided measurements of mass concentration (g/m3), or units which can be readily converted to mg/m3 (cf., Step S100). Hence, the exhaust flow can be combined with mass pollutant concentration (PC) on a mass/sec or mass/distance basis using the following equations:

Over a single measurement period (=1/F [s] where F is the measurement frequency in [Hz])

$$\text{Emissions} = (\text{EF } [m^3/s] \times \text{PC } [g/m^3]) / \text{Power, in } [g/kWs],$$

where Power is the average power [kW] during the measurement period (1/F [Hz]), or

$$\text{Emissions} = ((\text{Exhaust Flow } [m^3/s] \times \text{PC } [g/m^{3l}]) / \text{Distance, in } [g/km],$$

where Distance is the distance travelled during the measurement period 1/F [Hz]. For measurement frequencies other than 1Hz, the units will change accordingly.

Over a complete test cycle the total emissions can be calculated using the standard method of summing the individual readings and dividing by the total time elapsed or distance covered.

The above provided embodiment and in particular the formulas are given by way of example only and should not be viewed as limiting in any way. The mass emission rate estimation method of the present invention may also be based on different formulas or approximation approaches to estimate the volumetric exhaust flow based on vehicle mass and speed and additional optional factors to increase the estimation accuracy.

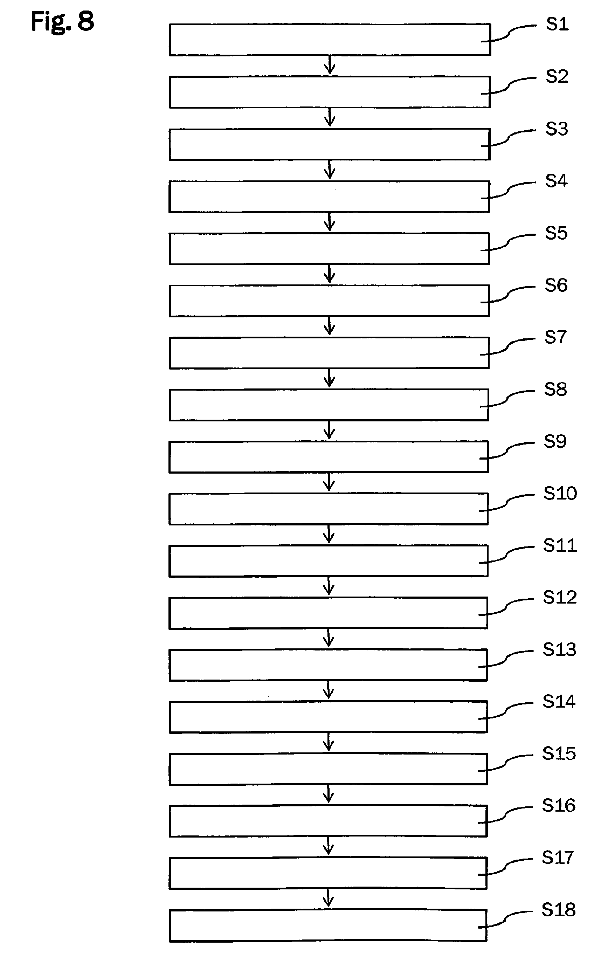

Fig. 8 shows a flow diagram of steps according to another embodiment of the present invention, including:

Step S1: measuring and recording the inertial mass of the vehicle being tested;

Step S2: driving the vehicle either on a dynamometer or on any fixed plane such as a road, either on a random driving pattern or a predetermined speed/time profile;

Step S3: whilst in motion, withdrawing a sample of exhaust from the raw exhaust stream and distributing the sample two instruments capable of continuously measuring each of the pollutants of interest;

Step S4: whilst in motion, continuously monitoring the speed of the vehicle;

Step S5: at a predetermined frequency recording the vehicle velocity, the engine revolutions rate and the concentration of each pollutant being measured;

Step S6: if it is also desired to measure the effect of gradient on pollutant emission rates, additionally and at the same frequency record the output of an inertia-based accelerometer aligned with the longitudinal axis of the vehicle under test;

Step S7: from the recorded vehicle velocity, for each frequency period, calculate the acceleration or deceleration rate of the vehicle;

Step S8: using empirical or measured data for frontal area, aerodynamic drag coefficient and tire rolling losses, calculate the instantaneous tractive force and power required to propel the vehicle at the current speed/acceleration rate combination for motion on a horizontal plane;

Step 9: the tractive force attributable to gradient may be determined by subtracting the previously calculated horizontal plane tractive force from the total inertial force determined by multiplying the vehicle mass by the rate of acceleration recorded by the accelerometer;

Step 10: from the instantaneous power and engine revolution rate calculate the instantaneous engine torque, taking account of a generic factor for power absorbed by the transmission and accessories driven by the engine;

Step 11: using an engine-specific or generic matrix of brake specific fuel consumption (BSFC) related to combinations of engine speed and torque, note the instantaneous mass rate of fuel consumption;

Step 12: using standard chemical constants, calculate the volume of carbon dioxide, corrected to normal temperature and pressure, generated in the current frequency period by stoichiometric combustion of the calculated fuel mass;

Step 13: using the known relative volumetric proportions of nitrogen and oxygen in ambient air, calculate the volumetric flow of ambient air which would be required for stoichiometric combustion of the calculated fuel mass burned in the current frequency period;

Step 14: compare the instantaneous concentration of carbon dioxide in the exhaust stream with the known concentration which would be present from stoichiometric combustion and correct the calculated volumetric flow of ambient air according to the ratio of theoretical carbon dioxide concentration and the actual measured carbon dioxide concentration;

Step 15 from the known relative volumetric proportions of nitrogen and oxygen in ambient air, calculate the instantaneous volumetric flow rate of nitrogen and add to this the calculated carbon dioxide volumetric flow rate, the result of which is the calculated total instantaneous volumetric exhaust flow;

Step 16: for each pollutant being measured, multiply the measured concentration, converted to grams per unit volume, by the calculated total instantaneous volumetric exhaust flow, corrected to normal temperature and pressure ;

Step 17: integrating this result for each frequency period, together with the actual distance travelled during each matching frequency period (determined from the instantaneous velocity measurement) allows simple calculation of the mass of each pollutant generated per kilometer of travel;

Step 18: similarly, by integrating the derived engine power at each frequency period with the mass of each pollutant emitted during each frequency period allows a second calculation of the mass of each pollutant generated per kilowatt hour.

Features, components and specific details of the structures of the above-described embodiments may be exchanged or combined to form further embodiments optimized for the respective application. As far as those modifications are readily apparent for an expert skilled in the art they shall be disclosed implicitly by the above description without specifying explicitly every possible combination, for the sake of conciseness of the present description.

## Claims

1.  A method of estimating a mass emission rate of a particulate or gaseous combustion product contained in the exhaust of a vehicle powered by a combustion-based energy source, comprising the steps of:

    measuring a combustion product concentration contained in the exhaust of the vehicle for a plurality of time intervals;
    determining a mass of the vehicle;
    measuring a velocity of travel of the vehicle for the plurality of time intervals;
    estimating a volumetric exhaust flow rate for the plurality of time intervals based on the determined mass and the velocity of travel; and
    calculating the mass emission rate of the pollutant as the product of the measured combustion product concentration and the determined volumetric exhaust flow rate.

2.  A method according to claim 1, further comprising the step of measuring a further combustion product concentration contained in the exhaust of the vehicle during each of the plurality of time intervals; wherein the determined further concentration is used to correct the estimated volumetric exhaust flow rate by a factor calculated as the ratio of the measured further combustion product concentration in the exhaust stream versus a concentration of the further combustion product concentration in the exhaust stream that would result from stoichiometric combustion.

3.  A method according to claim 2, wherein the measured further combustion product concentration is the carbon dioxide concentration.

4.  A method according to any of the preceding claims, wherein in the step of estimating a volumetric exhaust flow rate for the plurality of time intervals, an instantaneous power required to propel the vehicle is calculated for the plurality of time intervals based on the determined mass and the velocity of travel; and the calculated instantaneous power is used to calculated the instantaneous mass fuel consumption rate.

5.  A method according to any of the preceding claims, further comprising the step of determining a power-specific fuel consumption, wherein the power specific fuel consumption is determined either using an estimated constant brake specific fuel consumption value (BSFC) or using a BSFC value determined based on the measured engine speed and a determined engine torque during the time interval.

**6.** A method according to one of the preceding claims, wherein empirical or measured factors for at least one of an aerodynamic loss, a tyre rolling loss, a transmission loss or an accessory power loads is used to determine a tractive power required to propel the vehicle at the determined speed.

**7.** A method according to one of the preceding claims, wherein the output of a mass-based accelerometer aligned with the longitudinal axis of travel is used to determine a net tractive load due to the algebraic sum of a gradient and a longitudinal acceleration or deceleration.

**8.** A method according to one of the preceding claims, further comprising the step of integrating the estimated mass emission rates over the plurality of time intervals together with the actual distance travelled during the plurality of time intervals to calculate the mass of the combustion product generated per kilometer of travel.

**9.** A method according to one of the preceding claims, further comprising the steps of determining an engine power using a dynamometer for the plurality of time intervals and integrating the determined engine power with the estimated mass emission rates over the plurality of time intervals to calculate the mass of the pollutant generated per used energy unit.

**10.** A method according to one of the preceding claims, wherein the mass emission rate is estimated based on the driving of the vehicle on a normal road or on a dedicated test track.

**11.** A method according to one of the preceding claims, wherein the vehicle is tested on a dynamometer for estimating the mass emission rate, wherein different driving conditions are simulated using dynamometer load curves.

**12.** A system of estimating a mass emission rate of a particulate or gaseous pollutant contained in the exhaust of a vehicle powered by a combustion-based energy source, comprising:

> a processing unit;
> an emission analyzer device for measuring concentration rates of gaseous and particulate emissions connected to the processing unit;
> a sample hose transmitting an exhaust sample to the device for measuring concentration rates of gaseous and particulate emissions;
> **characterised in that** the processing unit is configured to conduct the method of estimating a mass emission rate according to any of the preceding claims 1 - 11.

**13.** A system according to claim 12, further comprising a vehicle velocity sensor and an engine speed sensor connected to the processing unit.

**14.** A system according to claim 12 or claim 13, wherein the emission analyzer device, and the vehicle velocity sensor are placed inside the vehicle during a measurement period for estimating the mass emission rate.

**Patentansprüche**

**1.** Verfahren zur Schätzung einer Massenemissionsrate eines Schwebstoffteilchens oder gasförmigen Verbrennungsprodukts, das im Abgas eines Fahrzeugs enthalten ist, welches durch eine auf Verbrennung basierende Energiequelle angetrieben wird, mit den folgenden Schritten.
Messen einer Verbrennungsproduktkonzentration, die im Abgas des Fahrzeugs enthalten ist, mehrere Zeitintervalle lang;
Bestimmen einer Masse des Fahrzeugs;
Messen einer Fahrgeschwindigkeit des Fahrzeugs die mehreren Zeitintervalle lang;
Schätzen einer volumetrischen Abgasströmungsrate die mehreren Zeitintervalle lang auf der Basis der bestimmten Masse und der Fahrgeschwindigkeit; und
Berechnen der Massenemissionsrate des Schadstoffs als Produkt der gemessenen Verbrennungsproduktkonzentration und der bestimmten volumetrischen Abgasströmungsrate.

**2.** Verfahren nach Anspruch 1, ferner mit dem Schritt des Messens einer weiteren Verbrennungsproduktkonzentration, die im Abgas des Fahrzeugs enthalten ist, während jedes der mehreren Zeitintervalle; wobei die bestimmte weitere Konzentration verwendet wird, um die geschätzte volumetrische Abgasströmungsrate um einen Faktor zu korrigieren, welcher berechnet wird als das Verhältnis der gemessenen weiteren Verbrennungsproduktkonzentration im Abgasstrom zu einer Konzentration der weiteren Verbrennungsproduktkonzentration im Abgasstrom, die sich aus einer stöchiometrischen Verbrennung ergeben würde.

3. Verfahren nach Anspruch 2, wobei die gemessene weitere Verbrennungsproduktkonzentration die Kohlendioxidkonzentration ist.

4. Verfahren nach irgendeinem der vorhergehenden Ansprüche, wobei im Schritt des Schätzens einer volumetrischen Abgasströmungsrate die mehreren Zeitintervalle lang eine sofortige Leistung, welche erforderlich ist, um das Fahrzeug anzutreiben, die mehreren Zeitintervalle lang auf der Basis der bestimmten Masse und der Fahrzeuggeschwindigkeit berechnet wird; und die berechnete sofortige Leistung verwendet wird, um die sofortige Massenkraftstoffverbrauchsrate zu berechnen.

5. Verfahren nach irgendeinem der vorhergehenden Ansprüche, ferner mit dem Schritt des Bestimmens eines leistungsspezifischen Kraftstoffverbrauchs, wobei der leistungsspezifische Kraftstoffverbrauch entweder unter Verwendung eines geschätzten kontanten bremsspezifischen Kraftstoffverbrauchswerts (brake specific fuel consumption, BSFC) oder unter Verwendung eines BSFC-Werts bestimmt wird, welcher auf der Basis der gemessenen Motordrehzahl und eines bestimmten Motordrehmoments während des Zeitintervalls bestimmt wird.

6. Verfahren nach einem der vorhergehenden Ansprüche, wobei empirische oder gemessene Faktoren für einen aerodynamischen Verlust und/oder einen Reifenrollverlust und/oder einen Übertragungsverlust und/oder eine akzessorische Leistungsladung verwendet wird, um eine Zugleistung zu bestimmen, welche erforderlich ist, um das Fahrzeug mit der bestimmten Geschwindigkeit anzutreiben.

7. Verfahren nach einem der vorhergehenden Ansprüche, wobei die Ausgabe eines massenbasierten Beschleunigungsmessers, der mit der Fahrlängsachse ausgerichtet ist, verwendet wird, um eine Nettozuglast aufgrund der algebraischen Summe eines Gradienten und einer Längsbeschleunigung oder -verzögerung zu bestimmen.

8. Verfahren nach einem der vorhergehenden Ansprüche, ferner mit dem Schritt des Integrierens der geschätzten Massenemissionsraten über die mehreren Zeitintervalle zusammen mit der Ist-Entfernung, die während der mehreren Zeitintervalle zurückgelegt wird, um die Masse des Verbrennungsprodukts zu berechnen, das pro Fahrkilometer erzeugt wird.

9. Verfahren nach einem der vorhergehenden Ansprüche, ferner mit den Schritten des Bestimmens einer Motorleistung unter Verwendung eines Dynamometers für die mehreren Zeitintervalle und des Integrierens der bestimmten Motorleistung mit den geschätzten Massenemissionsraten über die mehreren Zeitintervalle, um die Masse des pro verbrauchter Energieeinheit erzeugten Schadstoffs zu berechnen.

10. Verfahren nach einem der vorhergehenden Ansprüche, wobei die Massenemissionsrate auf der Basis des Fahrens des Fahrzeugs auf einer normalen Straße oder auf einer zweckbestimmten Teststrecke geschätzt wird.

11. Verfahren nach einem der vorhergehenden Ansprüche, wobei das Fahrzeug auf einem Dynamometer getestet wird, um die Massenemissionsrate zu schätzen, wobei unterschiedliche Fahrbedingungen unter Verwendung von Dynamometer-Lastkurven simuliert werden.

12. System zum Schätzen einer Massenemissionsrate eines Schwebstoffteilchens oder gasförmigen Schadstoffs, das/der im Abgas eines Fahrzeugs enthalten ist, welches von einer auf Verbrennung basierenden Energiequelle angetrieben wird, mit:

einer Verarbeitungseinheit;
einer Emissionsanalysatorvorrichtung zum Messen von Konzentrationsraten gasförmiger und Schwebstoffteilchenemissionen, die mit der Verarbeitungseinheit verbunden ist;
einem Probenschlauch, der eine Abgasprobe an die Vorrichtung zum Messen von Konzentrationsraten von gasförmigen und Schwebstoffteilchenemissionen überträgt;
**dadurch gekennzeichnet, dass** die Verarbeitungseinheit dazu konfiguriert ist, das Verfahren zum Schätzen einer Massenemissionsrate nach irgendeinem der vorhergehenden Ansprüche 1 bis 11 auszuführen.

13. System nach Anspruch 12, ferner mit einem Fahrzeuggeschwindigkeitssensor und einem Motordrehzahlsensor, die mit der Verarbeitungseinheit verbunden sind.

**14.** System nach Anspruch 12 oder Anspruch 13, wobei die Emissionsanalysatorvorrichtung und der Fahrzeuggeschwindigkeitssensor während einer Messperiode zum Schätzen der Massenemissionsrate im Fahrzeug platziert sind.

**Revendications**

**1.** Procédé pour estimer le taux d'émission massique d'un produit de combustion en forme de particules ou de forme gazeuse, qui est continu dans les gaz d'échappement d'un véhicule entraîné par une source d'énergie à base de combustion, comprenant les étapes suivantes :

- on mesure une concentration du produit de combustion contenue dans les gaz d'échappement du véhicule pour une pluralité d'intervalles de temps ;
- on détermine une masse du véhicule ;
- on mesure la vitesse de roulement du véhicule pour une pluralité d'intervalles de temps ;
- on estime le débit volumétrique des gaz d'échappement pour une pluralité d'intervalles de temps en se basant sur la masse déterminée et sur la vitesse de roulement ; et
- on calcule le taux d'émission massique des polluants à titre de produit de la concentration mesurée de produit de combustion et du débit volumétrique déterminé des gaz d'échappement.

**2.** Procédé selon la revendication 1, comprenant en outre l'étape consistant à mesurer une autre concentration de produit de combustion contenue dans les gaz d'échappement du véhicule pendant chacun de la pluralité d'intervalles de temps, l'autre concentration déterminée étant utilisée pour corriger le débit volumétrique estimé des gaz d'échappement par un facteur calculé à titre de relation de l'autre concentration mesurée du produit de combustion dans le flux de gaz d'échappement par rapport à une concentration à titre d'autre concentration de produit de combustion dans le flux de gaz d'échappement, qui résulterait d'une combustion stoechiométrique.

**3.** Procédé selon la revendication 2, l'autre concentration mesurée du produit de combustion est la concentration de dioxyde de carbone.

**4.** Procédé selon l'une quelconque des revendications précédentes, dans lequel, lors de l'étape d'estimation du débit volumétrique des gaz d'échappement pour une pluralité d'intervalles de temps, on calcule une puissance instantanée requise pour propulser le véhicule pour la pluralité d'intervalles de temps en se basant sur la masse déterminée et sur la vitesse de roulement, et on utilise la puissance instantanée calculée pour calculer le taux massique instantané de consommation de carburant.

**5.** Procédé selon l'une quelconque des revendications précédentes, comprenant en outre l'étape de détermination d'une consommation de carburant spécifique à la puissance, la consommation de carburant spécifique à la puissance étant déterminée soit en utilisant une valeur constante estimée de consommation de carburant spécifique au frein (BSFC) soit en utilisant une valeur BSFC déterminée basée sur le régime de moteur mesuré et sur un couple de rotation déterminé du moteur pendant l'intervalle de temps.

**6.** Procédé selon l'une des revendications précédentes, dans lequel des facteurs empiriques ou mesurés pour l'une au moins des pertes parmi la perte aérodynamique, la résistance au roulement des pneumatiques, une perte de transmission ou une charge de puissance d'accessoires sont utilisés pour déterminer une puissance de traction requise pour propulser le véhicule à une vitesse déterminée.

**7.** Procédé selon l'une des revendications précédentes, dans lequel la sortie d'un accéléromètre basé sur la masse, en alignement avec l'axe longitudinal de roulement, est utilisée pour déterminer une charge de traction nette à partir de la somme algébrique d'un gradient et d'une accélération ou décélération longitudinale.

**8.** Procédé selon l'une des revendications précédentes, comprenant en outre l'étape d'intégration des taux estimés d'émission massique sur la pluralité d'intervalles de temps ensemble avec la distance actuelle parcourue pendant la pluralité d'intervalles de temps en vue de calculer la masse du produit de combustion engendré par kilomètre de voyage.

9. Procédé selon l'une des revendications précédentes, comprenant en outre l'étape de détermination d'une puissance moteur en utilisant un dynamomètre pour la pluralité d'intervalles de temps et l'étape d'intégration de la puissance moteur déterminée avec les taux estimés d'émission massique sur la pluralité d'intervalles de temps en vue de calculer la masse de polluants engendrés par unité d'énergie utilisée.

10. Procédé selon l'une des revendications précédentes, dans lequel le taux d'émission massique est estimé en se basant sur le roulement du véhicule sur une route normale ou sur un parcours de test déterminé.

11. Procédé selon l'une des revendications précédentes, dans lequel le véhicule est testé sur un dynamomètre pour estimer le taux d'émission massique, différentes conditions de roulement étant simulées en utilisant des courbes de charge dynamométrique.

12. Système d'estimation d'un taux d'émission massique d'un polluant en forme de particules ou en forme gazeuse, qui est continu dans les gaz d'échappement d'un véhicule entraîné par une source d'énergie à base de combustion, comprenant :

   une unité de traitement ;
   un dispositif d'analyse d'émission pour mesurer les taux de concentration d'émissions en forme gazeuse et en forme de particules, qui est connecté à l'unité de traitement ;
   un tuyau de prise d'échantillon qui transmet un échantillon de gaz d'échappement au dispositif pour mesurer les taux de concentration des émissions en forme gazeuse ou en forme de particules ;
   **caractérisé en ce que** l'unité de traitement est configurée pour mettre en oeuvre le procédé d'estimation d'un taux d'émission massique selon l'une quelconque des revendications précédentes 1 à 11.

13. Système selon la revendication 12, comprenant en outre un capteur de vitesse de véhicule et un capteur de régime de moteur connectés à l'unité de traitement.

14. Système selon la revendication 12 ou la revendication 13, dans lequel le dispositif d'analyse d'émission et le capteur de vitesse de véhicule sont positionnés à l'intérieur du véhicule pendant une période de mesure en vue d'estimer le taux d'émission massique.

Fig. 1

EP 2 434 270 B1

# Fig. 2

**Fig. 3**

# Fig. 4

```
┌─────────────────────────────────────┐
│            Vehicle Mass              │ ──── S100
└─────────────────────────────────────┘
                   │
                   ▼
┌─────────────────────────────────────┐
│    Combustion Product Concentration  │ ──── S200
└─────────────────────────────────────┘
                   │
                   ▼
┌─────────────────────────────────────┐
│            Vehicle Speed             │ ──── S300
└─────────────────────────────────────┘
                   │
                   ▼
┌─────────────────────────────────────┐
│     Volumetric Exhaust Flow Rate     │ ──── S400
└─────────────────────────────────────┘
                   │
                   ▼
┌─────────────────────────────────────┐
│          Mass Emission Rate          │ ──── S500
└─────────────────────────────────────┘
```

# Fig. 5

```
┌─────────────────────────────┐
│        Inertia Load         │ ── S401
└─────────────────────────────┘
              │
              ▼
┌─────────────────────────────┐
│        Gradient Load        │ ── S402
└─────────────────────────────┘
              │
              ▼
┌─────────────────────────────┐
│      Aerodynamic  Drag      │ ── S403
└─────────────────────────────┘
              │
              ▼
┌─────────────────────────────┐
│        Rolling Losses       │ ── S404
└─────────────────────────────┘
              │
              ▼
┌─────────────────────────────┐
│        Tractive Load        │ ── S405
└─────────────────────────────┘
              │
              ▼
┌─────────────────────────────┐
│      Fuel Consumption       │ ── S406
└─────────────────────────────┘
              │
              ▼
┌─────────────────────────────┐
│     Fuel Volumetric Flow    │ ── S407
└─────────────────────────────┘
              │
              ▼
┌─────────────────────────────┐
│         Excess Air          │ ── S408
└─────────────────────────────┘
              │
              ▼
┌─────────────────────────────┐
│        Exhaust Flow         │ ── S409
└─────────────────────────────┘
```

Fig. 6

## Fig. 7A

TOTAL FLOW
12,17 l/s

N₂ = 9,49 l/s

O₂ = 2,55 l/s

Diesel 1.0 g/s

COMBUSTION

CO₂ = 1,8 l/s

TOTAL FLOW
11,29 l/s

CO2 Concentration = 15,93 %

## Fig. 7B

TOTAL FLOW
24,34 l/s

N₂ = 19,24 l/s

O₂ = 2,55 l/s

O₂ = 2,55 l/s

Diesel 1,0 g/s

COMBUSTION

O₂ = 2,55 l/s

CO₂ = 1,8 l/s

TOTAL FLOW
23,59 l/s

CO2 Concentration = 7,63 %

**Fig. 8**

S1

S2

S3

S4

S5

S6

S7

S8

S9

S10

S11

S12

S13

S14

S15

S16

S17

S18

**EP 2 434 270 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5639957 A
- WO 03038393 A1
- WO 2005085793 A1